# DEMANDE DE BREVET EUROPEEN

(11) **EP 2 962 755 A1**
(43) Date de publication de la demande: **06.01.2016**
(21) Numéro de dépôt: 15305859.9
(22) Date de dépôt: 05.06.2015
(51) Int. Cl.: B01J 31/14, B01J 31/38, C07C 2/26, C07C 11/08

(54) **COMPOSITION CATALYTIQUE ET PROCÉDÉ DE DIMERISATION SELECTIVE DE L'ETHYLENE EN BUTENE-1**

(30) Priorité: 04.07.2014 FR 1456471
(71) Demandeur: IFP Énergies nouvelles, 92852 Rueil-Malmaison Cedex (FR)
(72) Inventeur: Magna, Lionel, 69007 Lyon (FR); Olivier-Bourbigou, Helene, 69230 Saint Genis-Laval (FR)

(57) **Abrégé**

L'invention décrit une composition catalytique obtenue par l'interaction d'une part d'un titanate d'alkyle, avec d'autre part un mélange préformé d'un alkylaluminium et d'une base de Lewis. L'invention décrit également l'utilisation de ladite composition dans un procédé de dimérisation sélective de l'éthylène en butène-1.

## Description

La présente invention concerne une composition catalytique et un procédé de dimérisation sélective de l'éthylène en butène-1 utilisant ladite composition catalytique.

### Art Antérieur

Parmi les systèmes catalytiques capables de dimériser sélectivement l'éthylène en butène-1, il est possible d'identifier dans la littérature des systèmes catalytiques à base de vanadium (S. Zhang et al. Organometallics 2009, 28, 5925; K. Nomura et al. Inorg. Chem. 2013, 52, 2607), de fer ou de cobalt (S. Song et al. J. Organomet. Chem., 2011, 696, 2594; V. Appukuttan et al. Organometallics 2011, 30, 2285), de tungstène (H. Olivier et al. J. Mol. Catal. A: Chem. 1999, 148, 43; R. Tooze et al. Sasol Technology WO2005089940A2, 2005**),** de tantale (S. McLain et al. J. Am. Chem. Soc., 1978, 100(4), 1315; R. Schrock et al. Pure & App. Chem., 1980, 52, 729), de nickel (S. Mukherjee et al. Organometallics 2009, 28, 3074; K. Wang et al. Catal. Commun. 2009, 10, 1730; H. Liu et al. Dalton Trans. 2011, 40, 2614; J. Flapper et al. Organometallics 2009, 28, 3272, K. Song et al. Eur. J. Inorg. Chem. 2009, 3016) ou encore de titane (A. W. Al-Sa'doun, Applied Catalysis A: General, 1993, 105, 1-40).

Parmi ces systèmes, ceux à base de titane occupent de loin une position privilégiée. K. Ziegler décrit dans le brevet U.S 2.943.125, un procédé de dimérisation de l'éthylène en butène-1 au moyen d'un catalyseur obtenu par le mélange de trialkylaluminium et d'un tetraalcoolate de titane ou de zirconium. Lors de la réaction, se forme également une certaine quantité de polymère de haute masse moléculaire (à savoir le polyéthylène) qui gêne considérablement la mise en oeuvre du procédé. Plusieurs améliorations ont été proposées pour diminuer le taux de formation du polyéthylène, en particulier dans le brevet U.S. 3.686.350 qui préconise l'emploi de composés organiques du phosphore conjointement avec les éléments du catalyseur. Dans le brevet U.S. 3.879.485, une autre amélioration consiste à utiliser divers éthers comme solvants du milieu réactionnel. Bien que ces modifications du système catalytique initial apportent une amélioration substantielle à la sélectivité de la réaction, elles se révèlent d'une utilisation peu pratique, en particulier dans un procédé industriel dans lequel il faut pouvoir séparer le butène-1 du solvant en laissant seulement des traces de composé polaire dans les butènes.

De ce point de vue, le brevet FR 2 552 079 de la demanderesse a démontré que la mise en oeuvre d'un catalyseur obtenu par l'interaction d'un trialkylaluminium d'une part, avec d'autre part un mélange préformé de titanate d'alkyle et d'additif de type éther en quantité stoechiométrique, améliorait appréciablement l'activité et la sélectivité de ces catalyseurs pour la dimérisation de l'éthylène en butène-1. Des quantités de polymère faibles mais mesurables sont cependant détectées dans les exemples de ce brevet.

Le principal inconvénient des systèmes catalytiques à base de titane conduisant à la formation sélective de butène-1 est la formation en quantité non négligeable de polymères, à savoir le polyéthylène. Cette formation de polyéthylène peut être à l'origine d'une désactivation rapide du catalyseur et d'une difficulté accrue d'opérabilité.

Un objectif de l'invention est de fournir une composition catalytique particulière dont l'utilisation dans un procédé de dimérisation sélective de l'éthylène en butène-1 résulte en une production de polyéthylène réduite, voire quasi nulle par rapport à l'art antérieur.

### Description détaillée de l'invention

Les systèmes à base de titane décrit plus haut utilisent principalement comme co-catalyseur (ou activateur) un dérivé de type trialkylaluminium comme notamment le triéthylaluminium. Ce composé décrit dans la littérature comme une espèce dimérique (Al₂Et₆) se caractérise entre autre par une réactivité importante avec les composés de type base de Lewis (BL). Cette réaction, en générale fortement exothermique, conduit à la formation de nouveaux complexes d'aluminium stables pouvant très souvent être purifiés par distillation sous vide. Ces complexes sont en général monomériques de stoechiométrie BL/Al=1/1. Ils se présentent sous la forme de solide blanc ou de liquide incolore. Leurs propriétés physico-chimiques sont par ailleurs très différentes de celles du triéthylaluminium de départ. On peut citer à titre d'exemple, la diminution considérable de l'acidité de Lewis de ces complexes par rapport au triéthylaluminium de départ (Dailey et al., J. Am. Chem. Soc, 1956, 77, 3977 ; Y. Takashi, Bull. Chem. Soc. Jpn, 1967, 40, 612).

Il a maintenant été trouvé qu'une composition catalytique obtenue en mélangeant un composé alcoxy ou aryloxy du titane à un mélange préformé d'un additif de type base de Lewis et d'un composé d'aluminium dans des proportions voisines de la stoechiométrie, présente une sélectivité et une activité très élevées pour la dimérisation sélective de l'éthylène en butène-1 et ceci avec une production de polyéthylène réduite, voire nulle.

Sans vouloir être lié par une quelconque théorie, on peut imaginer que l'utilisation du mélange préformé d'un additif de type base de Lewis et d'un composé d'aluminium, permet au contact avec le composé du titane de limiter la génération d'espèces catalytiques responsables de la production de polymère.

Par « proportions voisines de la stoechiométrie ou quantité stoechiométrique », on entend que les additifs de type base de Lewis (BL) sont utilisés ou mélangés avec le composé d'aluminium (Al) dans un rapport molaire « BL/Al » strictement supérieur à 0,5, de préférence strictement supérieur à 1, de manière préférée compris entre une valeur strictement supérieure à 0,5 et 20, et de manière plus préférée compris entre une valeur strictement supérieure à 1 et 20. De préférence, le rapport molaire « BL/Al » est compris entre une valeur strictement supérieure à 0,5 et 5, de manière plus préférée compris entre une valeur strictement supérieure à 1 et 5.

Dans la suite du texte et dans ce qui précède, le rapport molaire entre l'additif de type base de Lewis (BL) et le composé d'aluminium (Al) sera, sauf indication contraire, exprimé en mole d'additif de type base de Lewis par mole d'aluminium (Al). De la même manière, le rapport molaire entre le composé d'aluminium et le composé alcoxy ou aryloxy du titane sera, sauf indication contraire, exprimé en mole d'aluminium par mole de titane.

On entend par base de Lewis toute entité moléculaire ou toute espèce chimique correspondante, en mesure de fournir une paire d'électrons et donc capable de se coordiner avec un acide de Lewis, produisant ainsi un adduit de Lewis.

Par « mélange préformé » on entend que l'additif de type base de Lewis et le composé d'aluminium sont mis en contact, séparément des autres composés de la composition catalytique, de manière préalable à leur utilisation dans la composition catalytique. De manière préférée, cette mise en contact se fait par addition d'une quantité stoechiométrique de l'additif de type base de Lewis sur le composé d'aluminium afin de former un adduit.

Les composés alcoxy du titane utilisés dans la présente invention répondent avantageusement à la formule générale [Ti(OR)₄] dans laquelle R est un radical alkyle linéaire ou ramifié comportant de 2 à 30 atomes de carbone. Le radical R peut comporter des substituants à base d'hétéroatome azoté, phosphoré, soufré et oxygéné.

Parmi les radicaux alcoxy préférés, on peut citer à titre d'exemple non limitatifs : le tétraéthoxy, tétraisopropoxy, le tétra-n-butoxy, le tétra-2-éthyl-hexyloxy.

Les composés aryloxy du titane utilisés dans la présente invention répondent avantageusement à la formule générale [Ti(OR')₄] dans laquelle R' est un radical aryle substitué ou non par des groupements alkyle, aryle ou aralkyle comportant de 2 à 30 atomes de carbone. Le radical R' peut comporter des substituants à base d'hétéroatome azoté, phosphoré, soufré et oxygéné.

Parmi les radicaux aryloxy préférés, on peut citer à titre d'exemple non limitatif : le phénoxy, le 2-méthylphénoxy, le 2,6-diméthylphénoxy, le 2,4,6-triméthylphénoxy, le 4-méthylphénoxy, le 2-phénylphénoxy, le 2,6-diphénylphénoxy, le 2,4,6-triphénylphénoxy, le 4-phénylphénoxy, le 2-tert-butyl-6-phénylphénoxy, le 2,4-ditertbutyl-6-phénylphénoxy, le 2,6-diisopropylphénoxy, le 2,6-ditert-butylphénoxy, le 4-méthyl-2,6-ditert-butylphénoxy, le 2,6-dichloro-4-tert-butylphénoxy et le 2,6-dibromo-4-tert-butylphénoxy, le radical biphénoxy, le binaphtoxy, le 1,8-naphtalène-dioxy.

Le composé d'aluminium selon l'invention est avantageusement choisi dans le groupe formé par les composés hydrocarbylaluminium, les composés tris(hydrocarbyl)aluminium, les composés chlorés ou bromés d'hydrocarbylaluminium et les aluminoxanes, de préférence ledit composé d'aluminium est avantageusement choisi dans le groupe formé par les composés hydrocarbylaluminium, les composés tris(hydrocarbyl)aluminium et les composés chlorés ou bromés d'hydrocarbylaluminium.

Les composés tris(hydrocarbyl)aluminiums et les composés chlorés ou bromés d'hydrocarbylaluminium sont représentés par la formule générale AIR"ₘY₃₋ₘ dans laquelle R" est un radical hydrocarbyle, de préférence alkyle comprenant de 1 à 6 atomes de carbone, Y est un atome de chlore ou de brome, de préférence un atome de chlore et m est un nombre de 1 à 3.

De préférence, le composé d'aluminium est choisi dans le groupe formé par le dichloroéthylaluminium (EtAlCl₂), le sesquichlorure d'éthylaluminium (Et₃Al₂Cl₃), le chlorodiéthylaluminium (Et₂AlCl), le chlorodiisobutylaluminium (*i*-Bu₂AlCl), le triéthylaluminium (AlEt₃), le tripropylaluminium (Al(*n*-Pr)₃), le triisobutylaluminium (Al(*i*-Bu)₃). Le composé d'aluminium préféré est le triéthylaluminium (AlEt₃).

L'additif de type base de Lewis de la composition catalytique selon l'invention est avantageusement choisi parmi les composés de type éther, amine, phosphine, sulfure, cycliques ou non cycliques, substitués ou non par des groupements alkyl, aryl, aralkyl comportant de 2 à 30 atomes de carbone.

Les composés de type éther sont avantageusement choisis parmi les monoéthers et les polyéthers. Parmi les composés de type éthers préférés, on peut citer à titre d'exemple non limitatifs : l'éther diéthylique, le diisopropyléther, le dibutyléther, le diphényléther le 2-méthoxy-2-méthylpropane, 2-methoxy-2-méthylbutane, le diméthoxy-2,2 propane, le di(éthyl-2 hexyloxy)-2,2 propane, le 2,5-dihydrofurane, le tétrahydrofurane, le 2-méthoxytétrahydrofurane, le 2-méthyltétrahydrofurane, le 3-méthyltétrahydrofurane, le 2,3-dihydropyrane, le tétrahydropyrane, le 1,3-dioxolane, le 1,3-dioxane, le 1,4-dioxane, le diméthoxyéthane, di(2-méthoxyéthyl)éther et le benzofurane, le glyme, le diglyme, pris seuls ou en mélange.

Les composés de type amine sont avantageusement choisis parmi les monoamines, les di-, tri- et poly-amines, les imines, les diimines, les pyridines, les bipyridines, les imidazoles, les pyrroles, les pyrazoles. Parmi les composés de type amines préférés, on peut citer à titre d'exemple non limitatifs : la triméthylamine, la triéthylamine, la pyridine, la 2-méthylpyridine, la 3-méthylpyridine, la 4-méthylpyridine, la 2-méthoxypyridine, la 3-méthoxypyridine, la 4-méthoxypyridine, la 2-fluoropyridine, la 3-fluoropyridine, la 3-triflurométhylpyridine, la 2-phénylpyridine, la 3-phénylpyridine, la 2-benzylpyridine, la 3,5-diméthylpyridine, la 2,6-diterbutylpyridine et la 2,6-diphénylpyridine, la quinoline, la 1,10-phénanthroline, N-méthylpyrrole, N-butylpyrrole N-méthylimidazole, le N-butylimidazole, la 2,2'-bipyridine, la N,N'-diméthyl-éthane-1,2-diimine, la N,N'-di-t-butyl-éthane-1,2-diimine, la N,N'-di-t-butyl-butane-2,3-diimine, la N,N'-diphényl-éthane-1,2-diimine, la N,N'-bis-(diméthyl-2,6-phényl)-éthane-1,2-diimine, la N,N'-bis-(diisopropyl-2,6-phényl)-éthane-1,2-diimine, la N,N'-diphényl-butane-2,3-diimine, la N,N'-bis-(diméthyl-2,6-phényl)-butane-2,3-diimine, la N,N'-bis-(diisopropyl-2,6-phényl)-butane-2,3-diimine.

Les composés de type phosphine sont avantageusement choisis parmi les phosphines, les polyphosphines, les oxydes de phosphines, les phosphites, les phosphonites et les phosphinites. Parmi les composés de type phosphine préférés, on peut citer à titre d'exemple non limitatifs : la tributylphosphine, la trisopropylphosphine, la tricyclohexylphosphine, la triphénylphosphine, la tris(o-tolyl)phosphine, le bis(diphénylphosphino)éthane, l'oxyde de trioctylphosphine, l'oxyde de triphénylphosphine, la triphénylphosphite.

Les composés de type sulfure sont avantageusement choisis parmi les mono-sulfures et les polysulfures. Parmi les composés de type sulfures préférés, on peut citer à titre d'exemple non limitatifs : le diéthylsulfure, le diméthyldisulfure, le tétrahydrothiophène, le 2-méthylthiophène, le 3-méthylthiophène, la 2-méthoxythiophène, la 3-méthoxythiophène.

Les additifs de type base de Lewis (BL) sont utilisés ou mélangés avec le composé d'aluminium (Al) dans un rapport molaire « BL/Al » strictement supérieur à 0,5, de préférence strictement supérieur à 1, de manière préférée compris entre une valeur strictement supérieure à 0,5 et 20, et de manière plus préférée compris entre une valeur strictement supérieure à 1 et 20. De préférence, le rapport molaire « BL/Al » est compris entre une valeur strictement supérieure à 0,5 et 5, de manière plus préférée compris entre une valeur strictement supérieure à 1 et 5.

Selon un mode de préparation préféré, le mélange préformé entre l'additif de type base de Lewis et le composé d'aluminium est réalisé par addition d'une quantité stoechiométrique de l'additif de type base de Lewis sur le composé d'aluminium. De préférence cette addition est réalisée en milieu dilué en utilisant un solvant. Dans ce cas, le solvant utilisé est avantageusement choisi dans le groupe formé par les hydrocarbures aliphatiques et cyclo-aliphatiques tels que l'hexane, le cyclohexane, l'heptane. Ce mélange est avantageusement réalisé à une température comprise entre -80°C et +200°C, de préférence entre -40°C et +100°C, par exemple à une température voisine de l'ambiante (15 à 30 °C).

Le composé alcoxy ou aryloxy de titane peut être utilisé en mélange avec un solvant de type hydrocarbure choisi dans le groupe formé par les hydrocarbures aliphatiques et cycloaliphatiques tels que l'hexane, le cyclohexane, l'heptane, le butane ou l'isobutane, par un hydrocarbure insaturé comme une monooléfine ou une dioléfine comportant par exemple de 4 à 20 atomes de carbone, par un hydrocarbure aromatique tel que le benzène, le toluène, l'ortho-xylène, le mésitylène, l'éthylbenzène, par un hydrocarbure chloré tel que le chlorobenzène ou le dichlorométhane, ou avec un solvant de type éther tel que le diméthyléther, le dibutyléther, le tétrahydrofuranne, le 1,4-dioxanne, purs ou en mélange. On utilise avantageusement les hydrocarbures aliphatiques comme le cyclohexane ou le n-heptane et les éthers comme le tétrahydrofuranne et le 1,4-dioxanne. Le mélange peut être effectué sous une atmosphère d'éthylène ou de gaz inerte.

Dans le cas où le composé alcoxy ou aryloxy de titane est utilisé en mélange avec un solvant de type hydrocarbure, ledit mélange est avantageusement utilisé dans un rapport volumique entre le solvant et le composé du titane compris entre 100/1 et 1/1 (vol/vol). Dans le cas où le composé alcoxy ou aryloxy de titane est utilisé en mélange avec un solvant de type éther, ledit mélange est avantageusement utilisé dans rapport molaire entre le solvant et le composé alcoxy ou aryloxy du titane compris entre 20/1 et 1/1 (mol/mol).

### Réaction de dimérisation :

Le procédé selon l'invention est un procédé de dimérisation sélective de l'éthylène en butène-1 mettant en oeuvre la composition catalytique décrite ci-dessus.

La réaction de dimérisation de l'éthylène est avantageusement effectuée sous une pression totale **de 0,5 à 20 MPa, de préférence** de 0,5 à 15 MPa, de préférence de 1 à 10 MPa, et à une température de 20 à 180°C, de préférence de 40 à 140°C.

Le ratio (ou rapport) molaire entre d'une part le mélange préformé comprenant le composé d'aluminium et l'additif de type base de Lewis, et d'autre part le composé alcoxy ou aryloxy du titane de la composition catalytique est mise en oeuvre tel que le rapport molaire entre le composé d'aluminium et le composé du titane est compris entre 1/1 et 1000/1 mol/mol, de préférence entre 1/1 et 500/1, de manière encore plus préférée, ce ratio est inférieur à 100/1 mol/mol.

La concentration du titane dans la solution catalytique est avantageusement comprise entre 1.10⁻⁹ et 1 mol/L, de préférence entre 1.10⁻⁶ et 0,5 mol/L.

Selon un mode de réalisation préféré, la réaction de dimérisation est mise en oeuvre en discontinu. On introduit des quantités choisies des solutions de composé du titane et du mélange préformé entre l'additif de type base de Lewis et le composé de l'aluminium, dans un réacteur muni des dispositifs habituels d'agitation, de chauffage et de refroidissement, puis on pressurise par de l'éthylène avantageusement à la pression désirée, et on ajuste la température avantageusement à la valeur souhaitée. Le réacteur de dimérisation est maintenu à pression constante par introduction d'éthylène jusqu'à ce que le volume total de liquide produit représente, par exemple, de 2 à 50 fois le volume de la solution comprenant la composition catalytique primitivement introduit. On détruit alors le catalyseur par tout moyen habituel connu de l'homme du métier, puis on soutire et on sépare les produits de la réaction et le solvant.

Selon un autre mode de réalisation préféré, la réaction catalytique de dimérisation de l'éthylène est mise en oeuvre en continu. Dans une première variante, on injecte séparément, dans un réacteur maintenu sous pression constante d'éthylène, d'une part le composé du titane et d'autre part le mélange préformé entre l'additif de type base de Lewis et le composé de l'aluminium. Ledit réacteur est agité par les moyens mécaniques classiques connus de l'homme du métier ou par une recirculation extérieure. La température et la pression d'éthylène sont maintenues constantes aux valeurs souhaitées en utilisant les moyens classiques connus de l'homme du métier. Le mélange réactionnel est soutiré au moyen d'une vanne asservie au niveau liquide de façon à maintenir celui-ci constant. Le catalyseur est détruit en continu par tout moyen habituel connu de l'homme du métier, puis les produits issus de la réaction ainsi que le solvant sont séparés, par exemple par distillation. L'éthylène qui n'a pas été transformé peut être recyclé dans le réacteur. Les résidus de catalyseur inclus dans une fraction lourde peuvent être incinérés.

Dans une seconde variante, on injecte dans un premier réacteur/mélangeur, d'une part le composé du titane, et d'autre part le mélange préformé entre l'additif de type base de Lewis et le composé d'aluminium, ledit mélange est ensuite introduit en continu dans un réacteur maintenu sous pression constante d'éthylène. Ledit mélange réalisé dans le premier réacteur/mélangeur peut être réalisée sous atmosphère inerte ou sous atmosphère d'éthylène. Le mélange réactionnel est soutiré au moyen d'une vanne asservie au niveau liquide de façon à maintenir celui-ci constant. Le catalyseur est détruit en continu par tout moyen habituel connu de l'homme du métier, puis les produits issus de la réaction ainsi que le solvant sont séparés, par exemple par distillation. L'éthylène qui n'a pas été transformé peut être recyclé dans le réacteur. Les résidus de catalyseur inclus dans une fraction lourde peuvent être incinérés.

### Produits obtenus :

Le procédé selon l'invention permet la production sélective de butène-1. Ce composé trouve une utilisation en tant que comonomères avec l'éthylène dans la fabrication de différents grades de polyéthylène (HDPE, LLDPE...).

Les exemples suivants illustrent l'invention sans en limiter la portée.

### Exemples

### Exemple 1 : Synthèse de l'adduit « THF.AlEt₃ »

Dans un schlenk sous atmosphère inerte, on introduit 25 mL de n-heptane dans lequel on dissout 0,832g g (7,3 mmol) de AlEt₃. On additionne ensuite de manière contrôlée 0,54 g (7,5 mmol) de THF. La solution est agitée à température ambiante durant environ 1 h. Le n-heptane est ensuite éliminé sous vide dynamique à température ambiante. L'adduit « THF.AlEt₃ » est isolé avec un rendement quasi quantitatif (95%) sous la forme d'un liquide incolore. Une analyse RMN ¹H permet de confirmer le ratio molaire THF/AlEt₃ à 1,03/1.

RMN ¹H: (300 MHz, CD₂Cl₂); δ(ppm): 4,12 (m, 4H); 2,11 (m, 4H); 1,04 (t, 9H); -0,18 (q, 6H).

### Exemples 2-10 :

Les essais de dimérisation de l'éthylène présentés dans le tableau 1 ci-dessous ont été réalisés dans un autoclave en acier inoxydable d'un volume utile de 500 mL, muni d'une double enveloppe permettant de réguler la température par circulation d'huile. L'agitation est assurée par une pale Rushton à entraînement mécanique. Dans ce réacteur on introduit sous atmosphère d'éthylène et à température ambiante, 40 mL de n-heptane ainsi que 5 mL d'une solution à 0,085 mol/L du composé de titane dans le n-heptane. Une fois la température du réacteur portée à 53°C, on introduit sous pression d'éthylène la quantité de co-catalyseur à base d'aluminium souhaité (préalablement dilué dans du n-heptane). Pour les exemples 2 à 4 (comparatifs), le co-catalyseur à base d'aluminium est AlEt₃. Pour les exemples 5 à 10 (conformes à l'invention), le co-catalyseur à base d'aluminium est l'adduit THF-AlEt₃ synthétisé à l'exemple 1. La pression d'éthylène est maintenue à 23 MPa et la température à 53°C. Après un temps « t » de réaction (voir tableau 1), l'introduction d'éthylène est arrêtée et le réacteur refroidi à 25°C. Le réacteur est ensuite dégazé au travers d'un compteur à gaz. Ce gaz est analysé par chromatographie en phase gazeuse. La phase liquide contenue dans le réacteur est ensuite pesée et analysée par chromatographie en phase gazeuse. Le polymère s'il est présent est récupéré, séché et pesé. La composition des produits obtenus est donnée dans le tableau 1 ci-après.

Dans le tableau 1, l'activité est définie comme la masse d'éthylène consommée par gramme de titane introduit initialement et par heure. Le %C₄ correspond à la quantité d'oléfines ayant un nombre d'atomes de carbone égal à 4 dans la distribution totale. Le %C₄⁼¹ représente la sélectivité en butène-1 dans la coupe C4. La quantité de polymère (%PE) correspond à la masse de polymère récupérée, ramenée à la distribution totale.

**Tableau 1:**

| Ex. | Composé de titane « Ti » | Co-catalyseur « Al » | Ratio Al/Ti | Temps (min) | Activité (g/gTi/h) | % C₄ (%C₄⁼¹) | %PE |
|---|---|---|---|---|---|---|---|
| | | | | | | | |
| 2 | [Ti(OⁿBu)₄] | AlEt₃ | 3 | 73 | 7400 | 91 (99⁺) | 0,79 |
| 3 | [Ti(OⁿBu)₄]/4THF** | AlEt₃ | 3 | 87 | 6200 | 94 (99⁺) | 0,05 |
| 4 | [Ti(OⁿBu)₄]/4THF** | AlEt₃ | 6.8 | 62 | 12400 | 94 (99⁺) | 0,30 |
| | | | | | | | |
| 5 | [Ti(OⁿBu)₄] | THF-AlEt₃ | 2 | 120 | 4100 | 95 (99+) | nd* |
| 6 | [Ti(OⁿBu)₄] | THF-AlEt₃ | 4 | 72 | 7900 | 94 (99+) | nd* |
| 7 | [Ti(OⁿBu)₄]/4THF** | THF-AlEt₃ | 2.5 | 94 | 5700 | 94 (99+) | nd* |
| 8 | [Ti(OⁿBu)₄]/4THF** | THF-AlEt₃ | 3 | 86 | 6300 | 94 (99+) | nd* |
| 9 | [Ti(OⁿBu)₄]/4THF** | THF-AlEt₃ | 4 | 79 | 7500 | 94 (99+) | nd* |
| 10 | [Ti(OⁿBu)₄]/4THF** | THF-AlEt₃ | 6 | 49 | 11000 | 92 (99+) | nd* |

| | | | | | | | |
|---|---|---|---|---|---|---|---|
| * nd= non détecté (cette dénomination caractérise l'absence totale de polymère). **Le composé du titane est utilisé en mélange avec le THF dans un rapport molaire THF/Ti de 4. | | | | | | | |

Dans ce tableau, les exemples 2 à 4 sont donnés à titre comparatif (non conforme à l'invention). Les exemples 5 à 10 montrent que l'utilisation d'un adduit préformé entre une base de Lewis, le THF en l'occurrence, et le triéthylaluminium selon l'invention permet d'obtenir de très bonnes activité et sélectivité pour la dimérisation sélective de l'éthylène en butène-1 tout en minimisant, jusqu'à un seuil inférieur à la limite de détection, la production de polyéthylène.

## Revendications

1. Composition catalytique comprenant au moins un composé alcoxy ou aryloxy du titane et au moins un mélange préformé entre un additif de type base de Lewis (BL) et un composé d'aluminium (Al) dans des proportions voisines de la stoechiométrie.

2. Composition catalytique selon la revendication 1 dans laquelle le rapport molaire « BL/Al » entre l'additif de type base de Lewis (BL) et le composé d'aluminium (Al) est strictement supérieur à 0,5.

3. Composition catalytique selon la revendication 1 ou 2 dans laquelle le rapport molaire entre le composé d'aluminium (Al) et le composé alcoxy ou aryloxy du titane est compris entre 1/1 et 1000/1 mol/mol.

4. Composition catalytique selon les revendications 1 à 3 dans laquelle les composés alcoxy du titane utilisés répondent à la formule générale [Ti(OR)₄] dans laquelle R est un radical alkyle linéaire ou ramifié comportant de 2 à 30 atomes de carbone.

5. Composition catalytique selon les revendications 1 à 3 dans laquelle les composés aryloxy du titane utilisés répondent à la formule générale [Ti(OR')₄] dans laquelle R' est un radical aryle substitué ou non par des groupements alkyle, aryle ou aralkyle comportant de 2 à 30 atomes de carbone.

6. Composition catalytique selon l'une des revendications précédentes dans laquelle le composé d'aluminium (Al) est choisi dans le groupe formé par les composés hydrocarbylaluminium, les composés tris(hydrocarbyl)aluminium, les composés chlorés ou bromés d'hydrocarbylaluminium et les aluminoxanes.

7. Composition catalytique selon l'une des revendications précédentes dans laquelle l'additif de type base de Lewis (BL) est choisi parmi les composés de type éther, amine, phosphine, sulfure, cycliques ou non cycliques, substitués ou non par des groupements alkyl, aryl, aralkyle comportant de 2 à 30 atomes de carbone.

8. Composition catalytique selon l'une des revendications précédentes dans laquelle le composé alcoxy ou aryloxy de titane est utilisé en mélange avec un solvant de type hydrocarbure choisi dans le groupe formé par les hydrocarbures aliphatiques et cyclo-aliphatiques, par un hydrocarbure insaturé ou une dioléfine, par un hydrocarbure aromatique, par un hydrocarbure, ou avec un solvant de type éther, purs ou en mélange.

9. Composition catalytique selon la revendication 8 dans laquelle lorsque le composé alcoxy ou aryloxy de titane est utilisé en mélange avec un solvant de type hydrocarbure, ledit mélange est avantageusement utilisé dans un rapport volumique entre le solvant et le composé du titane compris entre 100/1 et 1/1.

10. Composition catalytique selon la revendication 8 dans laquelle lorsque le composé alcoxy ou aryloxy de titane est utilisé en mélange avec un solvant de type éther, ledit mélange est utilisé dans rapport molaire entre le solvant et le composé du titane compris entre 20/1 et 1/1.

11. Composition catalytique selon l'une des revendications précédentes dans laquelle le mélange préformé entre l'additif de type base de Lewis (BL) et le composé d'aluminium (Al) est utilisé en mélange avec un solvant choisi dans le groupe formé par les hydrocarbures aliphatiques et cyclo-aliphatiques, par un hydrocarbure insaturé ou une dioléfine, par un hydrocarbure aromatique, par un hydrocarbure, purs ou en mélange.

12. Procédé de préparation de la composition catalytique selon l'une des revendications précédentes dans lequel on prépare le mélange préformé en additionnant une quantité stoechiométrique de l'additif de type base de Lewis (BL) sur le composé d'aluminium (Al), de préférence dans un milieu dilué en utilisant un solvant.

13. Procédé de préparation de la composition catalytique selon la revendication 12 dans lequel le mélange est réalisé à une température comprise entre -80°C et +200°C.

14. Procédé de dimérisation sélective de l'éthylène en butène-1 utilisant la composition catalytique selon l'une des revendications 1 à 11 ou susceptible d'être préparé selon l'une des revendications 12 ou 13.

15. Procédé de dimérisation sélective de l'éthylène en butène-1 selon la revendication 14 dans lequel la réaction de dimérisation est effectuée sous une pression totale de 0,5 à 20 MPa et à une température de 20 à 180°C.

16. Procédé de dimérisation sélective de l'éthylène en butène-1 selon la revendication 14 ou 15 dans lequel la réaction de dimérisation sélective de l'éthylène est mise en oeuvre dans un mode de réalisation discontinu ou continu.

17. Procédé de dimérisation sélective de l'éthylène en butène-1 selon l'une des revendications 14 à 16 mis en oeuvre tel que on introduit les quantités choisies des solutions de composé du titane et du mélange préformé entre l'additif de type base de Lewis et le composé de l'aluminium, dans un réacteur muni des dispositifs habituels d'agitation, de chauffage et de refroidissement, puis on pressurise par de l'éthylène, et on ajuste la température.

18. Procédé de dimérisation sélective de l'éthylène en butène-1 selon l'une des revendications 14 à 16 mis en oeuvre tel que on introduit séparément, dans un réacteur maintenue sous pression constante d'éthylène, d'une part le composé du titane et d'autre part le mélange préformé entre l'additif de type base de Lewis et le composé de l'aluminium.

19. Procédé de dimérisation sélective de l'éthylène en butène-1 selon l'une des revendications 14 à 16 mis en oeuvre tel que on introduit dans un premier réacteur/mélangeur, d'une part le composé du titane, et d'autre part le mélange préformé entre l'additif de type base de Lewis et le composé d'aluminium, ledit mélange étant ensuite introduit en continu dans un réacteur maintenue sous pression constante d'éthylène.
